(19) Europäisches Patentamt
European Patent Office
Office européen des brevets

(11) EP 3 094 248 B1

(12) **EUROPEAN PATENT SPECIFICATION**

(45) Date of publication and mention
of the grant of the patent:
**27.05.2020 Bulletin 2020/22**

(21) Application number: **15737500.7**

(22) Date of filing: **15.01.2015**

(51) Int Cl.:
*A61B 5/08* (2006.01)    *A61B 5/0205* (2006.01)

(86) International application number:
**PCT/FI2015/050023**

(87) International publication number:
**WO 2015/107268 (23.07.2015 Gazette 2015/29)**

(54) **METHOD AND DEVICE FOR THE DETECTION OF RESPIRATORY RATE**

VERFAHREN UND VORRICHTUNG ZUM NACHWEIS DER ATEMFREQUENZ

PROCÉDÉ ET DISPOSITIF DE DÉTECTION D'UNE FRÉQUENCE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.01.2014 FI 20145037**

(43) Date of publication of application:
**23.11.2016 Bulletin 2016/47**

(73) Proprietor: **Nokia Technologies Oy
02610 Espoo (FI)**

(72) Inventors:
• **OKSALA, Niku**
**FI-33700 Tampere (FI)**
• **LIUHANEN, Sasu**
**FI-02700 Kauniainen (FI)**

(74) Representative: **Whiting, Gary et al
Venner Shipley LLP
5 Stirling House
Stirling Road
The Surrey Research Park
Guildford GU2 7RF (GB)**

(56) References cited:
WO-A1-03/099114        WO-A1-2009/016334
WO-A1-2013/179018      US-A1- 2008 167 541
US-A1- 2012 296 219    US-A1- 2013 079 606
US-B1- 8 430 817

• CHON K. H. ET AL.: 'Estimation of Respiratory Rate From Photoplethysmogram Data Using Time-Frequency Spectral Estimation' IEEE TRANSACTIONS ON BIOMEDICAL ENGINEERING vol. 56, no. 8, 01 August 2009, PISCATAWAY, NJ, USA, XP011343010
• VENU M. K. ET AL.: 'Estimation of respiratory rate from principal components of photoplethysmographic signals' CONFERENCE PROCEEDING ARTICLE OF IEEE EMBS CONFERENCE ON BIOMEDICAL & SCIENCES. 30 November 2010 - 02 December 2010, pages 311 - 314, XP031936771
• NANDAKUMAR S. ET AL.: 'A Novel Approach Using Time Frequency Analysis of Pulse-Oximeter Data to Detect Progressive Hypovolemia in Spontaneously Breathing Healthy Subjects' IEEE TRANSACTIONS ON BIOMEDICAL ENGINEERING vol. 58, no. 8, 01 August 2011, XP011373099
• KARLEN, W. ET AL.: 'Respiratory rate estimation using respiratory sinus arrhythmia from photoplethysmography' CONFERENCE PROCEEDING ARTICLE OF 33RD ANNUAL INTERNATIONAL CONFERENCE OF THE IEEE EMBS 30 August 2011, pages 1201 - 1204, XP032318881

**Description**

TECHNICAL FIELD OF THE INVENTION

The invention relates to a method and device for the detection of respiratory rate. Especially the invention relates to continuous estimation of the respiratory rate.

BACKGROUND OF THE INVENTION

Respiratory rate is a sensitive surrogate of critical conditions that develop in patients hospitalized due to acute illness or healthy subjects developing life-threatening conditions. Respiratory rate is conventionally detected by physical examination, capnography, electrocardiography (ECG) based impedance variation, microphone-based technologies or by impedance tomography.

There are however some disadvantages related to the known techniques. Physical examination is not suitable for continuous monitoring, for example. Capnography requires wearing a mask, microphone-based technologies are sensitive to external noise and impedance tomography requires electrodes to be placed on the chest. These challenges limit the usability of existing technology especially for long-term monitoring of ambulatory patients or in sports or other personalized applications. WO2009/016334 discloses a method and apparatus for measuring breathing rate from a photoplethysmogram signal (PPG) by using auto-regressive modelling of the signal. The PPG signal is windowed in overlapping windows of typically 30 seconds' length, overlapping by 25 seconds, to obtain discrete sections of the signal and each section is low-pass filtered, downsampled and detrended and then AR modelled using an all-pole auto-regressive (AR) model. The AR model allows identification of the dominant frequencies in the signal and the pole corresponding to the breathing rate is identified by considering its magnitude and the breathing rate it represents.

SUMMARY OF THE INVENTION

An object of the invention is to alleviate and eliminate the problems relating to the known prior art. Especially the object of the invention is to provide a device for continuous monitoring of respiratory rate in a reliable, easy and fast way. The object of the invention can be achieved by the features of independent claims.

The invention relates to a method for estimating respiratory rate according to claim 1, as well as to a device for estimating respiratory rate according to claim 12 and computer program product of claim 15.

According to an embodiment a method for estimating respiratory rate comprises steps of:

- gathering data, such as spectrum data, related to peripheral pulse wave in an electrical form,
- extracting a good quality beat series with proper noise removal and interpolated beats for missing beats, which may comprise in an exemplary method extracting e.g. four primary sub-signals and two derived sub-signals from the beat series of said gathered data, which again can be used for selective averaging of said sub-signals,
- performing both time and frequency domain analysis independently for each of the aforementioned six sub-signals,
- combining the results of the time and frequency domain analysis independently for each sub-signal thus obtaining a group of estimates (2 x 6) for the respiratory rate,
- calculating the final estimate for the respiratory rate by removing sub-signal estimates with poor signal-to-noise ratio and those that are statistical outliers.

Optionally, the method may comprise also:

- determining averaged power spectrums and maximum power for each of said averaged power spectrums of said four sub-signals,
- combining said averaged power spectrums and maximums of said averaged power spectrums of said four sub-signals and determining a combined power spectrum,
- determining maximums in said combined power spectrum and frequency of sequence said determined maximums, and
- estimating respiratory rate based on said frequency determined from said sequential maximums of said combined power spectrum.

The (spectrum) data related to peripheral pulse wave is advantageously measured by peripheral sensors located for example at the wrist and fingers. The sensor may be for example a pulse oximeter, but also other suitable sensors can also be used, such as pressure sensors or the like.

An initial target of an embodiment of the invention is to obtain a reliable beat series by the following four steps, namely:

- pre-processing the spectrum data related to peripheral pulse wave, comprising e.g. a low pass filtering to remove high frequency noise,
- detection of peaks and troughs from the pre-processed data,
- filtering the peaks and troughs, and
- inter-beat-analysis with corrections for noise, ectopic and missing beats.

The obtained series is then used for the actual analysis, where the preliminary step is to extract new sub-signals comprising:

- amplitude (AM),
- baseline,
- inter-beat-interval (FM) (used as squared), and
- pulse width (used as squared).

Two composite signals are also created comprising:

- amplitude - baseline (phase difference), and
- inter-beat-interval + pulse width (used as squared).

Each sub-signal (n=6, for example) is resampled at constant intervals using cubic interpolation, as an example. Thereafter each sub-signal is subjected to both time and frequency domain analysis. Time domain analysis per signal comprises:

- peak and trough detection,
- detrending, and
- zero crossing detection.

An exemplary frequency estimate comprises the following steps:

1) f1 = ((n-peaks + n-troughs) / 2) / delta-time, and
2) f2 = (n-zero-crossings / 2) / delta-time

$$\text{Time domain estimate } f = \min(f1, f2),$$

when the two estimates differ, the difference is mostly caused by noise, whereupon the minimum is used.

An exemplary frequency domain analysis per signal comprises:

- windowing,
- FFT (Fast Fourier Transform),
- power spectrum calculation, and
- frequency estimation from the obtained power spectrum.

Signal-to-noise ratio is advantageously calculated for each time and frequency domain estimate. The time and frequency domain estimates of the sub-signals are then combined to calculate the final respiratory frequency estimate.

An exemplary combining the time and frequency domain estimates: comprises:

- 2 x (4 + 2) estimates,
- drop time domain estimates with poor estimated SNR (signal-to noise ratio), and
- drop frequency domain estimates with poor estimated SNR.

Use remaining estimates plus previous final estimate comprises:

- drop statistical outliers, and
- choose median = final estimate.

As in more details and according to an exemplary embodiment the method comprises the steps of low pass filtering the raw data. The data is detrended by subtracting 1s (example and configurable) average from each sample (detrended). The data is smoothed by calculating 1/5s (example and configurable) average and subtracting the 1s average (smoothed). The local minima and maxima are found by using both of the above mentioned (detrended and smoothed). For each minima and maxima of the smoothed curve (smoothed to reduce noise), the corresponding minima and maxima of the detrended data are detected and used to create beat candidates.

The exemplary method comprises also filter for the beat candidates as follows:

> if multiple candidates exist for one minimum / maximum of the averaged curve, select the best:

> - if no statistical data exists:
>   ◦ calculate a score based on distance between the smoothed and the detrended miminum / maximum + peripheral flow index (pfi),
> - otherwise:
>   ◦ use a weighted average of the following standard deviations:
>     ▪ pulse width, amplitude, pfi, distance of the maximum and distance of the minimum

Next, inter-beat-interval analysis may be used for the remaining candidates as following:

- if a candidate is statistically an ectopic beat:
  ◦ remove the candidate and replace it with a synthetic beat (location midway between previous and following) (width average of the previous and following)
- if a beat is statistically missed (either truly missed or missed during analysis)
  ◦ add a synthetic beat (see above)
- if a beat is statistically noise, mark it noise

A corrected beat series is thus obtained and used for further analysis.

The present invention offers advantages over the known prior art, such as very reliable measuring results due to using four sub-signals in the estimation process. In addition the invention offers possibility to use signals from a number of different sensors simultaneously and for same estimation process. Moreover the data processing steps of the raw data as described is not very vulnerable to environmental noise, for example. Especially it is to be noted that the current invention enables continuous estimation process for the respiratory rate.

BRIEF DESCRIPTION OF THE DRAWINGS

Next the invention will be described in greater detail with reference to exemplary embodiments in accordance with the accompanying drawings, in which:

Figure 1A      illustrates a principle of an exemplary method for estimating respiratory rate according to an advantageous embodiment of the invention,

Figure 1B      illustrates a principle of another exemplary method for estimating respiratory rate according to an advantageous embodiment of the invention,

Figure 2      illustrates an exemplary raw data gathered by a measuring device, such as pulse oximeter, according to an advantageous embodiment of the invention,

Figures 3A-3D      illustrate examples of resampled and extracted sub-samples used for estimating respiratory rate according to an advantageous embodiment of the invention and

Figure 4      illustrates an exemplary device and arrangement for estimating respiratory rate according to an advantageous embodiment of the invention.

DETAILED DESCRIPTION

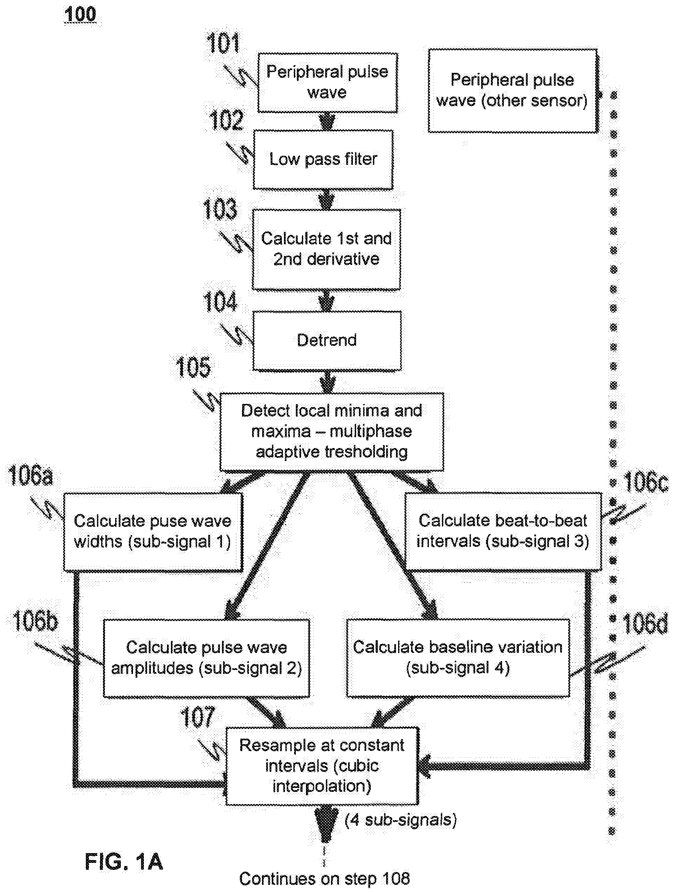

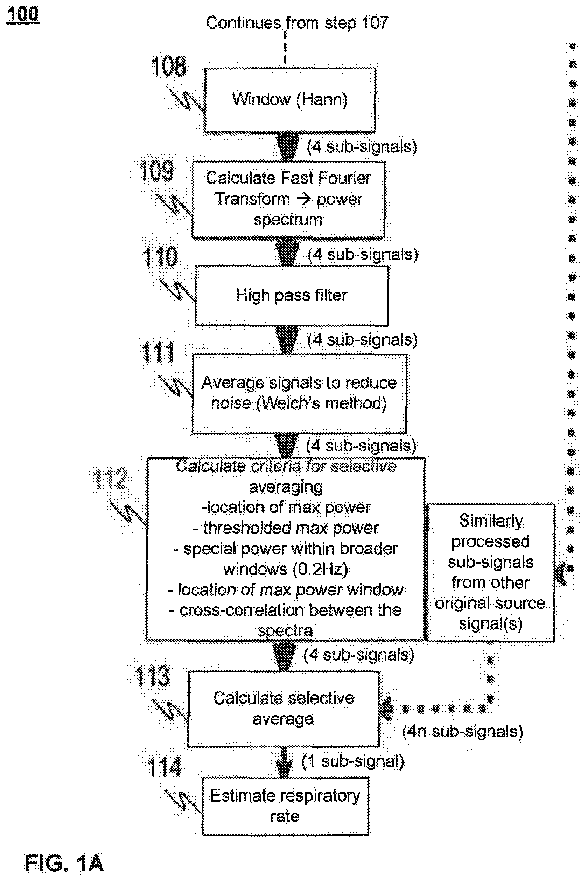

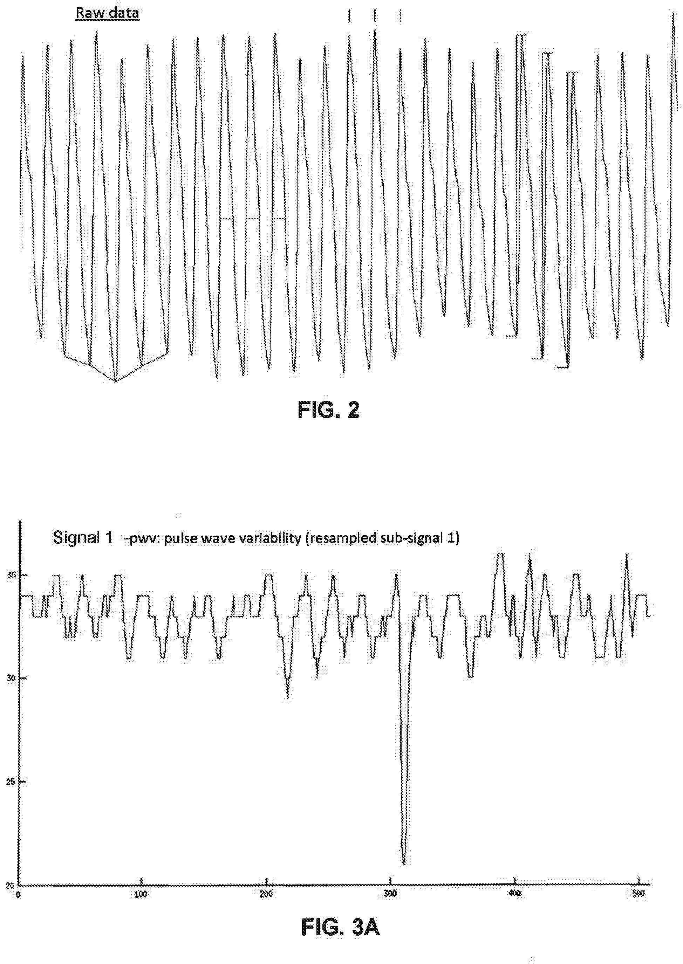

Referring to the Figure 1A the method 100 for estimating respiratory rate according to an advantageous embodiment of the invention comprises the following steps. According to an embodiment of the invention the respiratory rate of a patient is determined based on the signals measured 101 by peripheral sensors located for example at the wrist and fingers. The device for the detection of the respiratory rate of a patient advantageously continuously and non-invasively comprises either a photo-plethysmography (PPG) sensor, an infrared (IR) sensor, charge coupled device (CCD), optically by CMOS sensor, pulse oximeter or pressure sensor, such as an Electromechanical film (EMFi) sensor or a capacitive pressure sensor, tonometer or impedance or dielectric spectroscopy configured to record pulse wave as a raw data at the wrist or finger. It is to be noted that plurality of different sensors can be used, and that plurality of different raw data signals can be provided. An example of the raw data is described in Figure 2.

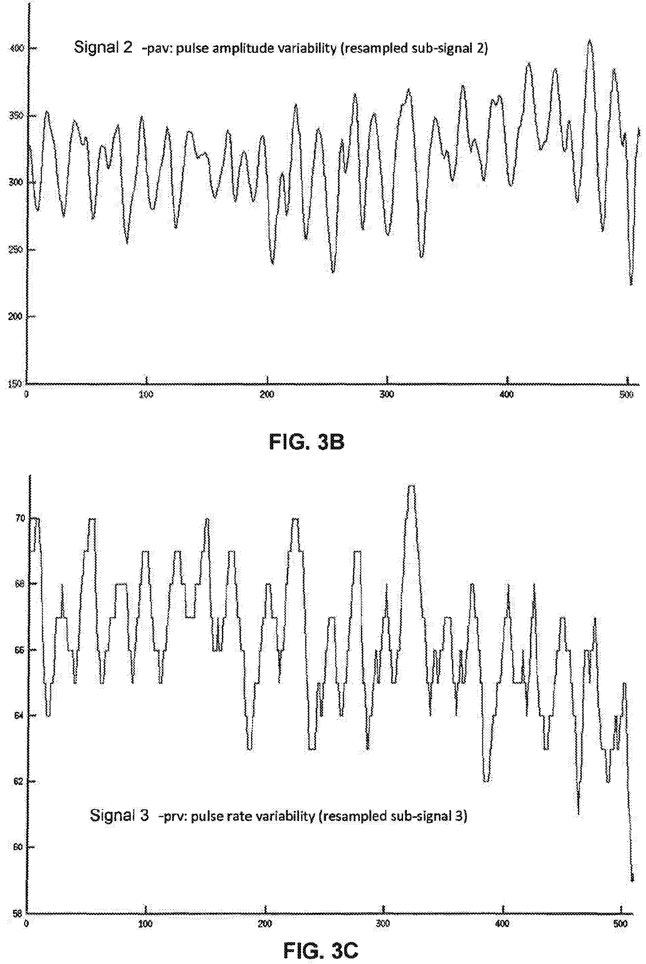

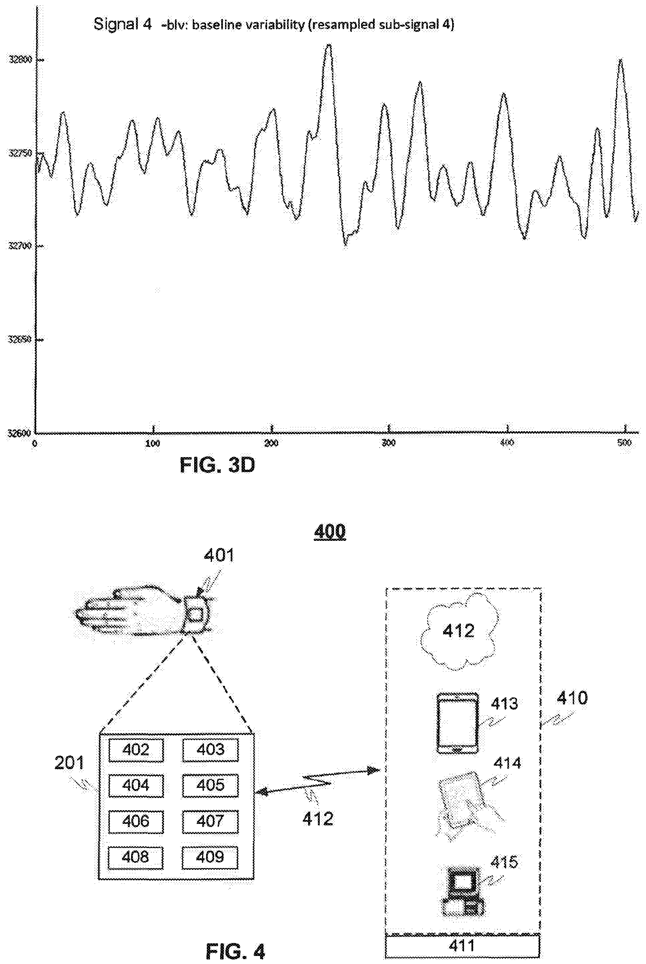

According to an embodiment low-pass filtering (e.g. 5 Hz) is performed 102 first to the measured raw data and the first and second derivatives of the resulting low-pass filtered signal are calculated 103. The low-pass filtering is done for removing high-frequency artefacts. The signal is then detrended 104 to remove any DC-component. The local minima and maxima are identified 105 from the detrended signal using the previously calculated derivatives and multi-phase adaptive thresholding is performed to identify peaks and troughs of true pulse waves. Thereafter, four sub-signals are calculated 106a, 106b, 106c, 106d from (each) raw data signal. Examples of derived four sub-signals are described in Figures 3A-3D.

For these four sub-signals the width of the pulse waves is calculated at first ((sub signal 1) 106a). The onset and end of each pulse wave can be detected using the previously calculated derivatives and set of rules based on their behaviour. Second, the pulse wave amplitudes are calculated ((sub signal 2) 106b); amplitude modulation of the original signal. Third, pulse rate variability is calculated ((sub signal 3) 106c); frequency modulation of the original signal. And fourth, the degree of baseline variation is calculated ((sub signal 4) 106d). Each of the four sub-signals is then resampled 107 at constant intervals using cubic interpolation. The sub-signals are Hann-windowed 108 and subjected to Fast Fourier Transformation (FFT) 109. The results are high-pass filtered 110 in order to eliminate low frequency bands and a power spectrum is obtained for each. Welch's method 111 is used to reduce noise. The maximum power is estimated per spectrum. It is to be noted that the order of determining said sub-signals can vary.

In the advantageous embodiment of the invention all these four sub-signals are used for determining the respiratory rate. In practice it might be that one of these four sub-signal might not be reliable readable, whereupon the respiratory rate may either be derived using at least three of those. Additionally readability of these four sub-signals can be used as a quality check for reliability of the derived respiratory rate.

According to an embodiment the sub-signal related to the pulse wave amplitudes or changes of the pulse wave amplitudes is processed also separately for determining a volemia (condition of the volume of plasma or blood circulating in the body). Namely, as an example, if the sub-signal related to the pulse wave amplitudes or changes of the pulse wave amplitudes of the pulse wave diminish (or disappears), the volume of plasma or blood circulating in the body is very high and reliability of the measurements of the respiratory rate by the method degrades. According to an example this can be used as a quality control check for the reliability of the measurements.

Additional criteria are calculated 112 using the four spectra: frequency corresponding to the maximum power per spectrum, thresholded maximum power per spectrum, maximum power within broader, clinically relevant 0.2 Hz frequency windows per spectrum, occurrence of maximum power within these windows and the cross-correlation between the spectra. Using these criteria, the four power spectra are selectively averaged and maximum power of the averaged spectrum is located 113. As an end result, the system provides an estimation of the respiratory rate 114.

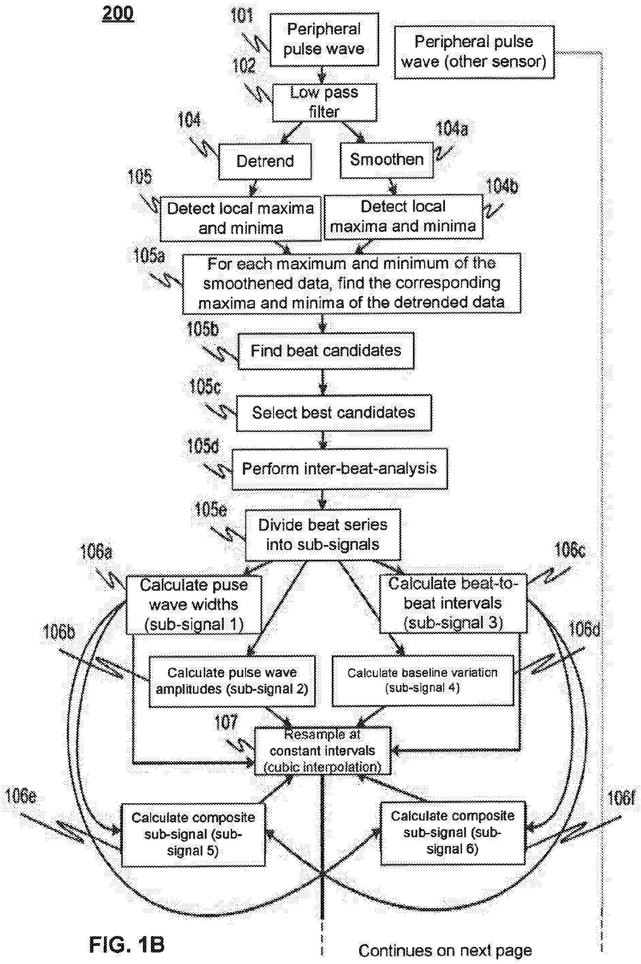

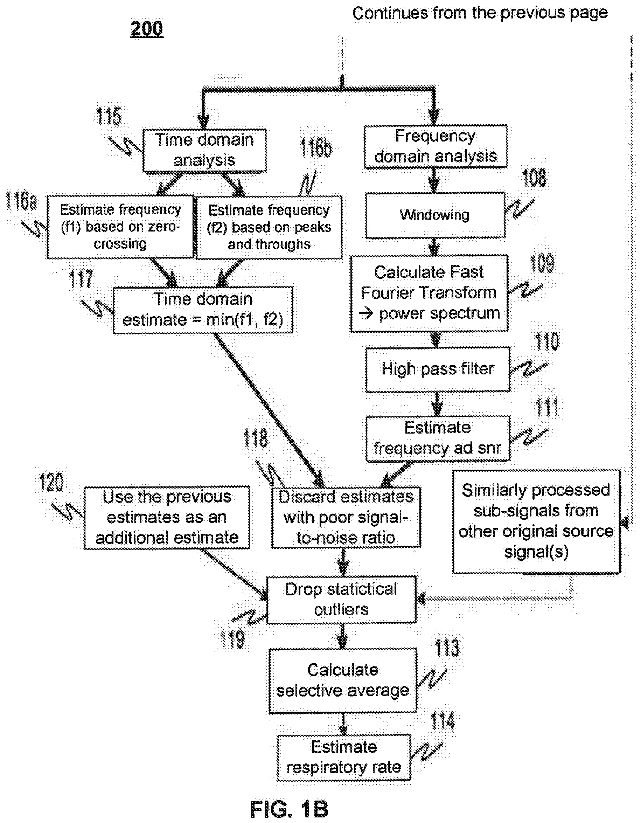

Figure 1B illustrates a principle of another exemplary method 200 for estimating respiratory rate according to an advantageous embodiment of the invention, which advantageously comprises number of similar and same features and step with the method 100 described in Figure 1A, such as especially steps 101-105, 106a-106d, 107-111 and 113-114. The method 200 describes an additional or alternative step 104a for smoothening the loss pass filtered data and step 104b for detecting local maxima and minima of the smoothed data. In addition the method 200 describes the steps after 105 in more details, where in step 105a the corresponding maxima and minima of the detrended data is found for each maximum and minimum of the smoothed data. The in step 105b beat candidates are found, in step 105c the best candidates are selected, in step 105d inter-beat-analysis is performed and in step 105e beat series is divided into sub-signals.

The method 200 also comprises steps 106e and 106f for calculating composite sub-signals (sub-signal 5 and sub-signal 6).

After resampling of the sub-signals 107 the method continues to steps for two alternative routes, either via step 108a of frequency domain analysis to steps 108-111, or via step 115 of time domain analysis to steps 116a and/or 116b of estimating frequency (f1) based on zero-crossing and/or of estimating frequency (f2) peaks and troughs. The next step 117 in this route is to perform and set time domain estimate as min(f1, f2).

The branches of the method 200 continue in step 118, where estimates with poor signal-to-noise ratio is discarded and step 119 where statistical outliers are dropped, and where the previous estimate can be used as an additional estimate 120. After this the method is continued as in example of Figure 1A with steps 113 and 114.

Figure 4 illustrates an exemplary device 401 and arrangement 400 for estimating respiratory rate according to an advantageous embodiment of the invention, where the device 401 comprises a data gathering means 402 for gathering a spectrum data related to pulse wave in an electrical form, and a data processing means 403 configured for:

- extracting four sub-signals of said spectrum data for selective averaging of said four sub-signals,
- determining averaged power spectrums and maximum power for each of said averaged power spectrums of said four sub-signals,
- combining said averaged power spectrums and maximums of said averaged power spectrums of said four sub-signals and determining a combined power spectrum,
- determining maximums in said combined power spectrum and frequency of sequence said determined maximums, and
- estimating respiratory rate based on said frequency determined from said sequential maximums of said combined power spectrum.

Advantageously the device 401 comprises or said data gathering means for gathering a spectrum data related to pulse wave in an electrical form comprises a photo-plethysmography (PPG) sensor 404, an infrared (IR) sensor 405, charge coupled device (CCD) 406, optically by CMOS sensor, pulse oximeter 407, or pressure sensor, such as an Electromechanical film (EMFi) sensor or a capacitive pressure sensor 408, tonometer or impedance or dielectric spectroscopy configured to record said pulse wave as a raw data. According to an example the device 401 is implemented as a wristband device with possible auxiliary means, whereupon the sensors to be used are advantageously configured to be positioned at the wrist or finger of the patient the respiratory rate of which is to be estimated.

However, it is to be noted that the data processing can be implemented by the wristband device by the data processing means 403, or alternatively the wristband device may send (e.g. wireless way) the measuring signals to the external data processing backend 410 for data calculation, which comprises the data processing means 411. Thus the data processing means 403 is optional in the device 401. For communicating 412 with the backend 410, the device 401 advantageously comprises data communication device 409, most advantageously wireless communication device, such as implemented by the Bluetooth or the like known by a skilled person.

The data processing backend 410 may comprise e.g. cloud server 412, any computer or mobile phone application 413-415 and according to an example it can send the calculated results or otherwise processed data e.g. for displaying back to the wristband device or other data displaying device, such as a computer or the like in data communication network or to a smartphone of the user.

The invention has been explained above with reference to the aforementioned embodiments, and several advantages of the invention have been demonstrated. It is clear that the invention is not only restricted to these embodiments, but comprises all possible embodiments within the scope of the following patent claims.

## Claims

1. A method for estimating respiratory rate,
   wherein the method comprises steps of:

   - gathering data related to a pulse wave in an electrical form,
   - extracting a beat series with noise removal and interpolated beats for missing beats,
   - extracting (106) one of

      (i) four primary sub-signals from the beat series and performing frequency domain analysis independently for each of the aforementioned at least four sub-signals, and
      (ii) four primary sub-signals and two derived sub-signals from the beat series and performing both time and frequency domain analysis independently for each of the aforementioned six sub-signals, wherein the four primary sub-signals comprise a first sub-signal indicative of pulse wave widths of the pulse wave; a second sub-signal indicative of an amplitude of the pulse wave; a third sub-signal indicative of an inter-beat time interval; and a fourth signal indicative of a degree of baseline variation in the pulse wave; and wherein the two derived sub-signals comprise a fifth sub-signal comprising the difference between the second sub-signal and the fourth sub-signal and a sixth sub-signal comprising sum of the first sub-signal and the third sub-signal;

      - combining, for at least three of the four primary sub-signals, the results of the frequency domain analysis for each sub-signal or the results of the time and frequency domain analysis for each of the six sub-signals, the combining comprising determining a group of estimates for the respiratory rate, the group of estimates comprising estimates of the respiratory rate determined using each of the sub-signals independently of the others;
      - calculating the final estimate (114) for the respiratory rate by removing sub-signal estimates with poor signal-to-noise ratio and those that are statistical outliers.

**2.** A method of claim 1, wherein the method comprises:

- determining averaged power spectrums and maximum power for each of said averaged power spectrums of said four sub-signals,
- combining said averaged power spectrums and maximums of said averaged power spectrums of said four sub-signals and determining a combined power spectrum,
- determining maximums in said combined power spectrum and frequency of a sequence of said determined maximums, and
- estimating respiratory rate based on said frequency determined from said sequential maximums of said combined power spectrum.

**3.** A method of claim 2, wherein said determining averaged power spectrums comprises averaging of each of said four sub-signals spectrums, and wherein said selective averaging comprises:

- determining location of maximum power,
- thresholding maximum power per sub-signal to identify peaks and troughs of true pulse waves,
- determining spectral power within predetermined windows,
- determining location of maximum power window,
- determining maximum power within maximum power window, and
- determining cross-correlation between the spectra.

**4.** A method of any of the previous claims, wherein the spectrum data is gathered by at least one peripheral sensor located at the wrist and finger area.

**5.** A method of any of the previous claims, wherein said gathered spectrum data is at first preprocessed by removing high-frequency artefacts using a low pass filter.

**6.** A method of any of the previous claims, wherein a first and second derivate are determined from said gathered spectrum data, and said data is detrended to remove a DC-component, and local minima and maxima are identified from said detrended signal using the previously calculated derivatives.

**7.** A method of any of the previous claims, wherein before the combination each of said four sub-signals is resampled at constant intervals using cubic interpolation, Hann-windowed, subjected to Fast Fourier Transformation (FFT), high-pass filtered to eliminate low frequency bands and thereby deriving a maximum power spectrum for each of said four sub-signals.

**8.** A method of any of the previous claims, wherein Welch's method is used to reduce noise from data.

**9.** A method of any of the previous claims, wherein said four sub-signals comprises:

- a first signal relating to width of the pulse wave,
- a second signal relating to pulse wave amplitude,
- a third signal relating to pulse rate variability, and
- a fourth signal relating to degree of baseline variation.

**10.** A method of any of the previous claims, wherein one of the sub-signals relates to pulse wave amplitudes and the sub-signal related to pulse wave amplitudes or changes of the pulse wave amplitudes of the pulse wave is processed for determining a volemia and thereby reliability of the measurements so that if the sub-signal related to the width of the pulse wave diminish below a threshold value, the volume of plasma or blood circulating in the body is determined to be very high and reliability of the measurements of the respiratory rate is determined to be invalid.

**11.** A method of any of the previous claims, wherein said estimation process is continuous and non-invasive estimation process for the respiratory rate.

**12.** A device for estimating respiratory rate,
wherein the device comprises:

- data gathering means for gathering data related to a pulse wave in an electrical form,

- data processing means configured for:

 o gathering data related to the pulse wave in an electrical form,
 o extracting a beat series with noise removal and interpolated beats for missing beats,
 o extracting (106) one of:

  (i) four primary sub-signals from the beat series and performing frequency domain analysis independently for each of the aforementioned at least four sub-signals, and
  (ii) four primary sub-signals and two derived sub-signals from the beat series and performing both time and frequency domain analysis independently for each of the aforementioned six sub-signals, wherein the four primary sub-signals comprise a first sub-signal indicative of pulse wave widths of the pulse wave; a second sub-signal indicative of an amplitude of the pulse wave; a third sub-signal indicative of an inter-beat time interval; and a fourth signal indicative of a degree of baseline variation in the pulse wave; and wherein the two derived sub-signals comprise a fifth sub-signal comprising the difference between the second sub-signal and the fourth sub-signal and a sixth sub-signal comprising sum of the first sub-signal and the third sub-signal;

 ◦ combining, for at least three of the four primary sub-signals, the results of the frequency domain analysis independently for each sub-signal or the results of the time and frequency domain analysis independently for each of the six sub-signals, the combining comprising determining a group of estimates for the respiratory rate, the group of estimates comprising estimates of the respiratory rate using each of the sub-signals independently of the others;
 ◦ calculating a final estimate (114) for the respiratory rate by removing sub-signal estimates with poor signal-to-noise ratio and those that are statistical outliers.

13. A device of claim 12, wherein the device is configured to process the sub-signal related to pulse wave amplitudes or changes of the pulse wave amplitudes of the pulse wave for determining a volemia and thereby reliability of the measurements so that if the sub-signal related to the width of the pulse wave diminish below a threshold value, the volume of plasma or blood circulating in the body is determined to be very high and reliability of the measurements of the respiratory rate is determined to be invalid.

14. A device of any of the previous device claims 12-13, wherein the device comprises a photo-plethysmography (PPG) sensor, an infrared (IR) sensor, charge coupled device (CCD), a CMOS sensor configured to record the pulse wave optically, pulse oximeter, or pressure sensor, such as an Electromechanical film (EMFi) sensor or a capacitive pressure sensor, tonometer or impedance or dielectric spectroscopy configured to record said pulse wave as a raw data, and wherein said sensor is configured to be positioned at the wrist or finger of the patient the respiratory rate of which is to be estimated.

15. A computer program product for estimating respiratory rate, **characterized in that** it comprises program code means stored on a computer-readable medium, which code means are arranged to perform all the steps of the method defined in claims 1 -10, when the program is run on the device of any of claims 12-14.

**Patentansprüche**

1. Verfahren zum Schätzen der Atemfrequenz,
wobei das Verfahren die Schritte umfasst:

 - Erfassen von Daten mit Bezug auf eine Pulswelle in einer elektrischen Form,
 - Extrahieren einer Schlagserie mit Geräuschentfernung und interpolierten Schlägen für fehlende Schläge,
 - Extrahieren (106) von einem von

  (i) vier primären Subsignalen aus der Schlagserie und unabhängiges Durchführen einer Frequenzdomänenanalyse für jedes der vorgenannten mindestens vier Subsignale, und
  (ii) vier primären Subsignalen und zwei abgeleiteten Subsignalen aus der Schlagserie und unabhängiges Durchführen von sowohl Zeit- als auch Frequenzdomänenanalyse für jedes der vorgenannten sechs Subsignale, wobei die vier primären Subsignale ein erstes Subsignal umfassen, das Pulswellenbreiten der Pulswelle angibt; ein zweites Subsignal, das eine Amplitude der Pulswelle angibt; ein drittes Subsignal, das ein Schlagabstandszeitintervall angibt; und ein viertes Signal, das einen Grad von Baseline-Abweichung in der Pulswelle angibt; und wobei die zwei abgeleiteten Subsignale ein fünftes Subsignal umfassen, das die Differenz zwischen dem zweiten Subsignal und dem vierten Subsignal umfasst, und ein sechstes Subsignal, das die Summe des ersten Subsignals und des dritten Subsignals umfasst;

- Kombinieren, für mindestens drei der vier primären Subsignale, der Resultate der Frequenzdomänenanalyse für jedes Subsignal oder der Resultate der Zeit- und Frequenzdomänenanalyse für jedes der sechs Subsignale, wobei das Kombinieren das Ermitteln einer Gruppe von Schätzwerten für die Atemfrequenz umfasst, wobei die Gruppe von Schätzwerten Schätzwerte der Atemfrequenz umfasst, die unter Verwendung von jedem der Subsignale unabhängig von den anderen ermittelt wurden;
- Berechnen des finalen Schätzwertes (114) für die Atemfrequenz mittels Entfernens von Subsignal-Schätzwerten mit schlechtem Signal-RauschVerhältnis und derjenigen, die statistische Ausreißer sind.

2. Verfahren nach Anspruch 1, wobei das Verfahren umfasst:

- Festlegen der gemittelten Leistungsspektren und der maximalen Leistung für jedes der gemittelten Leistungsspektren der vier Subsignale,
- Kombinieren der gemittelten Leistungsspektren und Maxima der gemittelten Leistungsspektren der vier Subsignale und Festlegen eines kombinierten Leistungsspektrums,
- Festlegen der Maxima in dem kombinierten Leistungsspektrum und einer Frequenz einer Sequenz der festgelegten Maxima, und
- Schätzen der Atemfrequenz basierend auf der aus den sequenziellen Maxima des kombinierten Leistungsspektrums festgelegten Frequenz.

3. Verfahren nach Anspruch 2, wobei das Festlegen der gemittelten Leistungsspektren die Durchschnittsbildung jedes der vier Subsignalspektren umfasst und wobei die selektive Durchschnittsbildung umfasst:

- Ermitteln der Position der maximalen Leistung,
- Schwellenwertbildung maximaler Leistung pro Subsignal zum Identifizieren von Peaks und Senken echter Pulswellen,
- Ermitteln von Spektralleistung in vorbestimmten Fenstern,
- Ermitteln der Position des maximalen Leistungsfensters,
- Ermitteln der maximalen Leistung in dem maximalen Leistungsfenster und
- Feststellen von Kreuzkorrelation zwischen den Spektren.

4. Verfahren nach einem der vorstehenden Ansprüche, wobei die Spektrumsdaten durch mindestens einen im Bereich von Handgelenk und Fingern positionierten Sensor erfasst werden.

5. Verfahren nach einem der vorstehenden Ansprüche, wobei die erfassten Spektrumsdaten zunächst mittels Entfernens hochfrequenter Artefakte unter Verwendung eines Tiefpassfilters vorverarbeitet werden.

6. Verfahren nach einem der vorstehenden Ansprüche, wobei eine erste und zweite Derivation aus den erfassten Spektrumsdaten ermittelt werden und die Daten trendbereinigt werden, um eine DC-Komponente zu entfernen, und lokale Minima und Maxima aus dem trendbereinigten Signal unter Verwendung der zuvor berechneten Derivationen identifiziert werden.

7. Verfahren nach einem der vorstehenden Ansprüche, wobei vor der Kombination jedes der vier Subsignale in konstanten Intervallen unter Verwendung kubischer Intervention neu abgetastet, Hann-gefenstert, einer schnellen Fourier-Transformation (FFT) unterzogen, hochpassgefiltert wird, um Niederfrequenzbänder zu eliminieren und dadurch ein maximales Leistungsspektrum für jedes der vier Subsignale abzuleiten.

8. Verfahren nach einem der vorstehenden Ansprüche, wobei zum Reduzieren von Rauschen aus Daten das Welsh-Verfahren eingesetzt wird.

9. Verfahren nach einem der vorstehenden Ansprüche, wobei die vier Subsignale umfassen:

- ein erstes Signal mit Bezug auf die Breite der Pulswelle,
- ein zweites Signal mit Bezug auf die Amplitude der Pulswelle,

- ein drittes Signal mit Bezug auf die Variabilität der Pulswelle,
- ein viertes Signal mit Bezug auf den Grad der Baseline-Abweichung.

10. Verfahren nach einem der vorstehenden Ansprüche, wobei sich eines der Subsignale auf Pulswellenamplituden bezieht und das Subsignal mit Bezug auf Pulswellenamplituden oder Änderungen der Pulswellenamplituden verarbeitet wird, um eine Volämie und somit die Zuverlässigkeit der Messungen zu bestimmen, sodass, falls das Subsignal mit Bezug auf die Breite der Pulswelle unter einen Schwellenwert abnimmt, das Volumen von in dem Körper zirkulierendem Plasma oder Blut als sehr hoch bestimmt wird und die Zuverlässigkeit der Messungen der Atemfrequenz als ungültig festgestellt wird.

11. Verfahren nach einem der vorstehenden Ansprüche, wobei der Schätzprozess ein kontinuierlicher und nicht invasiver Schätzprozess der Atemfrequenz ist.

12. Einrichtung zum Schätzen der Atemfrequenz,
    wobei die Einrichtung umfasst:

    - Datenerfassungsmittel zum Erfassen von Daten mit Bezug auf eine Pulswelle in einer elektrischen Form,
    - Datenverarbeitungsmittel, ausgelegt zum

        ◦ Erfassen von Daten mit Bezug auf eine Pulswelle in einer elektrischen Form,
        ◦ Extrahieren einer Schlagserie mit Geräuschentfernung und interpolierten Schlägen für fehlende Schläge,
        ◦ Extrahieren (106) von einem von

            (i) vier primären Subsignalen aus der Schlagserie und unabhängiges Durchführen einer Frequenzdomänenanalyse für jedes der vorgenannten mindestens vier Subsignale, und
            (ii) vier primären Subsignalen und zwei abgeleiteten Subsignalen aus der Schlagserie und unabhängiges Durchführen von sowohl Zeitals auch Frequenzdomänenanalyse für jedes der vorgenannten sechs Subsignale, wobei die vier primären Subsignale ein erstes Subsignal umfassen, das Pulswellenbreiten der Pulswelle angibt; ein zweites Subsignal, das eine Amplitude der Pulswelle angibt; ein drittes Subsignal, das ein Schlagabstandszeitintervall angibt; und ein viertes Signal, das einen Grad von Baseline-Abweichung in der Pulswelle angibt; und wobei die zwei abgeleiteten Subsignale ein fünftes Subsignal umfassen, das die Differenz zwischen dem zweiten Subsignal und dem vierten Subsignal umfasst, und ein sechstes Subsignal, das die Summe des ersten Subsignals und des dritten Subsignals umfasst;

        ◦ unabhängigen Kombinieren, für mindestens drei der vier primären Subsignale, der Resultate der Frequenzdomänenanalyse für jedes Subsignal oder der Resultate der Zeit- und Frequenzdomänenanalyse für jedes der sechs Subsignale unabhängig voneinander, wobei das Kombinieren das Ermitteln einer Gruppe von Schätzwerten für die Atemfrequenz umfasst, wobei die Gruppe von Schätzwerten Schätzwerte der Atemfrequenz umfasst, die unter Verwendung von jedem der Subsignale unabhängig von den anderen ermittelt wurden;
        ◦ Berechnen eines finalen Schätzwertes (114) für die Atemfrequenz mittels Entfernens von Subsignal-Schätzwerten mit schlechtem Signal-Rausch-Verhältnis und denjenigen, die statistische Ausreißer sind.

13. Einrichtung nach Anspruch 12, wobei die Einrichtung dafür ausgelegt ist, das Subsignal mit Bezug auf Pulswellenamplituden oder Änderungen der Pulswellenamplituden zu verarbeiten, um eine Volämie und somit die Zuverlässigkeit der Messungen zu bestimmen, sodass, falls das Subsignal mit Bezug auf die Breite der Pulswelle unter einen Schwellenwert abnimmt, das Volumen von in dem Körper zirkulierendem Plasma oder Blut als sehr hoch bestimmt wird und die Zuverlässigkeit der Messungen der Atemfrequenz als ungültig festgestellt wird.

14. Einrichtung nach einem der vorstehenden Einrichtungsansprüche 12-13, wobei die Einrichtung einen Photoplethysmografie-(PPG)-Sensor, einen Infrarot-(IR)-Sensor, eine ladungsgekoppelte Einrichtung (CCD), einen zum optischen Aufzeichnen der Pulswelle ausgelegten CMOS-Sensor, ein Pulsoximeter oder einen Drucksensor, wie einen elektromechanischen Film-(EMFi)-Sensor oder einen kapazitiven Drucksensor, ein Tonometer oder Impedanz- oder dielektrische Spektroskopie umfasst, ausgelegt zum Aufzeichnen der Pulswelle als Rohdaten, und wobei der Sensor dafür ausgelegt ist, an dem Handgelenk oder Finger des Patienten, dessen Atemfrequenz geschätzt werden soll, positioniert zu werden.

**15.** Rechnerprogrammprodukt zum Schätzen einer Atemfrequenz, **dadurch gekennzeichnet, dass** es auf einem rechnerlesbaren Medium gespeicherte Programmcodemittel umfasst, wobei die Codemittel eingerichtet sind, um alle Schritte des in den Ansprüchen 1-10 definierten Verfahrens durchzuführen, wenn das Programm auf der Einrichtung nach einem der Ansprüche 12-14 ausgeführt wird.

**Revendications**

1. Procédé permettant d'estimer une fréquence respiratoire,
   le procédé comprenant les étapes comprenant :

   - le rassemblement de données relatives à une onde de pouls sous forme électrique,
   - l'extraction d'une série de battements avec suppression du bruit et battements interpolés pour les battements manquants,
   - l'extraction (106) d'un parmi

     (i) quatre sous-signaux primaires provenant de la série de battements et effectuant une analyse de domaine fréquentiel indépendamment pour chacun des au moins quatre sous-signaux susmentionnés, et
     (ii) quatre sous-signaux primaires et deux sous-signaux dérivés de la série de battements et effectuant une analyse de domaine temporel et de domaine fréquentiel à la fois, indépendamment pour chacun des six sous-signaux susmentionnés, dans lequel les quatre sous-signaux primaires comprennent un premier sous-signal indiquant des largeurs d'onde de pouls de l'onde de pouls ; un deuxième sous-signal indiquant une amplitude de l'onde de pouls ; un troisième sous-signal indiquant un intervalle temporel entre les battements ; et un quatrième signal indiquant un degré de variation de ligne de référence dans l'onde de pouls ; et dans lequel les deux sous-signaux dérivés comprennent un cinquième sous-signal comprenant la différence entre le deuxième sous-signal et le quatrième sous-signal et un sixième sous-signal comprenant la somme du premier sous-signal et du troisième sous-signal ;

     - la combinaison, pour au moins trois des quatre sous-signaux primaires, des résultats de l'analyse de domaine fréquentiel pour chaque sous-signal ou des résultats de l'analyse de domaine temporel et de domaine fréquentiel pour chacun des six sous-signaux, la combinaison comprenant la détermination d'un groupe d'estimations pour la fréquence respiratoire, le groupe d'estimations comprenant des estimations de la fréquence respiratoire déterminée à l'aide de chacun des sous-signaux indépendamment des autres ;
     - le calcul de l'estimation finale (114) pour la fréquence respiratoire en supprimant des estimations de sous-signaux ayant un faible rapport signal sur bruit et ceux qui sont des valeurs aberrantes statistiques.

2. Procédé selon la revendication 1, le procédé comprenant :

   - la détermination de spectres de puissance moyennés et d'une puissance maximum pour chacun desdits spectres de puissance moyennés desdits quatre sous-signaux,
   - la combinaison desdits spectres de puissance moyennés et des maximums desdits spectres de puissance moyennés desdits quatre sous-signaux et la détermination d'un spectre de puissance combiné,
   - la détermination de maximums dans ledit spectre de puissance combiné et d'une fréquence d'une séquence desdits maximums déterminés, et
   - l'estimation d'une fréquence respiratoire sur la base de ladite fréquence déterminée à partir desdits maximums séquentiels dudit spectre de puissance combiné.

3. Procédé selon la revendication 2, dans lequel ladite détermination de spectres de puissance moyennés comprend le calcul de la moyenne de chacun des spectres desdits quatre sous-signaux, et dans lequel ledit calcul sélectif de la moyenne comprend :

   - la détermination de l'emplacement d'une puissance maximum,
   - le seuillage d'une puissance maximum par sous-signal pour identifier des crêtes et des creux d'ondes de pouls vraies,
   - la détermination d'une puissance spectrale à l'intérieur de fenêtres prédéfinies,
   - la détermination d'un emplacement d'une fenêtre de puissance maximum,
   - la détermination d'une puissance maximum à l'intérieur d'une fenêtre de puissance maximum, et
   - la détermination d'une corrélation croisée entre les spectres.

4. Procédé selon l'une quelconque des revendications précédentes, dans lequel les données de spectre sont rassemblées par au moins un capteur périphérique situé au niveau de la zone du poignet et des doigts.

5. Procédé selon l'une quelconque des revendications précédentes, dans lequel lesdites données de spectre rassemblées sont tout d'abord prétraitées en éliminant les artefacts haute fréquence à l'aide d'un filtre passe-bas.

6. Procédé selon l'une quelconque des revendications précédentes, dans lequel une première et une seconde dérivée sont déterminées à partir desdites données de spectre rassemblées, et lesdites données sont dissociées pour éliminer une composante c.c., et les minimums et maximums locaux sont identifiés à partir dudit signal dissocié à l'aide des dérivées précédemment calculées.

7. Procédé selon l'une quelconque des revendications précédentes, dans lequel, avant la combinaison, chacun desdits quatre sous-signaux est rééchantillonné à intervalles constants à l'aide d'une interpolation cubique, est soumis à une fenêtre de Hann, est soumis à une transformée de Fourier rapide (FFT), est soumis à un filtrage passe-haut afin d'éliminer les bandes basse fréquence, ce qui permet de dériver un spectre de puissance maximum pour chacun desdits quatre sous-signaux.

8. Procédé selon l'une quelconque des revendications précédentes, dans lequel la méthode de Welch est utilisée pour réduire un bruit à partir de données.

9. Procédé selon l'une quelconque des revendications précédentes, dans lequel lesdits quatre sous-signaux comprennent :

   - un premier signal relatif à une largeur de l'onde de pouls,
   - un deuxième signal relatif à une amplitude de l'onde de pouls,
   - un troisième signal relatif à une variabilité de la fréquence de pouls, et
   - un quatrième signal relatif à un degré de variation de ligne de référence.

10. Procédé selon l'une quelconque des revendications précédentes, dans lequel un des sous-signaux concerne des amplitudes d'onde de pouls et le sous-signal concernant des amplitudes d'onde de pouls ou des variations des amplitudes d'onde de pouls de l'onde de pouls est traité pour déterminer une volémie et de ce fait la fiabilité des mesures de sorte que, si le sous-signal concernant la largeur de l'onde de pouls passe sous une valeur de seuil, le volume de plasma ou de sang circulant dans l'organisme soit déterminé comme étant très élevé et que la fiabilité des mesures de la fréquence respiratoire soit déterminée comme étant non valide.

11. Procédé selon l'une quelconque des revendications précédentes, dans lequel ledit procédé d'estimation est un procédé d'estimation en continu et non invasif pour la fréquence respiratoire.

12. Dispositif permettant d'estimer une fréquence respiratoire,
    le dispositif comprenant :

    - des moyens de rassemblement de données pour rassembler des données relatives à une onde de pouls sous forme électrique,
    - des moyens de traitement de données configurés pour :

       ◦ le rassemblement de données relatives à l'onde de pouls sous forme électrique,
       ◦ l'extraction d'une série de battements avec suppression du bruit et battements interpolés pour les battements manquants,
       ◦ l'extraction (106) d'un parmi :

          (i) quatre sous-signaux primaires provenant de la série de battements et réalisant une analyse de domaine fréquentiel indépendamment pour chacun des au moins quatre sous-signaux susmentionnés, et
          (ii) quatre sous-signaux primaires et deux sous-signaux dérivés de la série de battements et effectuant une analyse de domaine temporel et de domaine fréquentiel à la fois, indépendamment pour chacun des six sous-signaux susmentionnés, dans lequel les quatre sous-signaux primaires comprennent un premier sous-signal indiquant des largeurs d'onde de pouls de l'onde de pouls ; un deuxième sous-signal indiquant une amplitude de l'onde de pouls ; un troisième sous-signal indiquant un intervalle temporel entre les battements ; et un quatrième signal indiquant un degré de variation de ligne de référence dans l'onde de pouls ; et dans lequel deux sous-signaux dérivés comprennent un cinquième sous-signal comprenant la différence entre le deuxième sous-signal et le quatrième sous-signal et un sixième sous-signal comprenant la somme du premier sous-signal et du troisième sous-signal ;

◦ la combinaison, pour au moins trois des quatre sous-signaux primaires, des résultats de l'analyse de domaine fréquentiel indépendamment pour chaque sous-signal ou des résultats de l'analyse de domaine temporel et de domaine fréquentiel indépendamment pour chacun des six sous-signaux, la combinaison comprenant la détermination d'un groupe d'estimations pour la fréquence respiratoire, le groupe d'estimations comprenant des estimations de la fréquence respiratoire à l'aide de chacun des sous-signaux indépendamment des autres ;

◦ le calcul d'une estimation finale (114) pour la fréquence respiratoire en supprimant des estimations de sous-signal ayant un faible rapport signal sur bruit et celles qui sont des valeurs aberrantes statistiques.

13. Dispositif selon la revendication 12, le dispositif étant configuré pour traiter le sous-signal concernant des amplitudes d'onde de pouls ou des variations des amplitudes d'onde de pouls de l'onde de pouls pour déterminer une volémie et de ce fait la fiabilité des mesures de sorte que, si le sous-signal concernant la largeur de l'onde de pouls passe sous une valeur de seuil, le volume de plasma ou de sang circulant dans l'organisme soit déterminé comme étant très élevé et que la fiabilité des mesures de la fréquence respiratoire soit déterminée comme étant non valide.

14. Dispositif selon l'une quelconque des revendications 12 et 13 précédentes, le dispositif comprenant un capteur photopléthysmographique (PPG), un capteur infrarouge (IR), un dispositif à couplage de charge (CCD), un capteur CMOS configuré pour enregistrer optiquement l'onde de pouls, un oxymètre de pouls, ou un capteur de pression, tel qu'un capteur à film électromécanique (EMFi) ou un capteur de pression capacitif, un tonomètre ou une spectroscopie d'impédance ou diélectrique configurée pour enregistrer ladite onde de pouls en tant que données brutes, et dans lequel ledit capteur est conçu pour être positionné au niveau du poignet ou d'un doigt du patient dont la fréquence respiratoire doit être estimée.

15. Produit-programme informatique permettant l'estimation d'une fréquence respiratoire, **caractérisé en ce qu'**il comprend des moyens de code de programme mémorisés sur un support lisible par ordinateur, lesquels moyens de code sont conçus pour exécuter toutes les étapes du procédé selon les revendications 1 à 10, lorsque le programme est exécuté sur le dispositif selon l'une quelconque des revendications 12 à 14.

**100**

101 — Peripheral pulse wave

Peripheral pulse wave (other sensor)

102 — Low pass filter

103 — Calculate 1st and 2nd derivative

104 — Detrend

105 — Detect local minima and maxima – multiphase adaptive tresholding

106a — Calculate puse wave widths (sub-signal 1)

106c — Calculate beat-to-beat intervals (sub-signal 3)

106b — Calculate pulse wave amplitudes (sub-signal 2)

106d — Calculate baseline variation (sub-signal 4)

107 — Resample at constant intervals (cubic interpolation)

(4 sub-signals)

**FIG. 1A**

Continues on step 108

**100**

Continues from step 107

108 — Window (Hann)

(4 sub-signals)

109 — Calculate Fast Fourier Transform → power spectrum

(4 sub-signals)

110 — High pass filter

(4 sub-signals)

111 — Average signals to reduce noise (Welch's method)

(4 sub-signals)

112 — Calculate criteria for selective averaging
-location of max power
- thresholded max power
- special power within broader windows (0.2Hz)
- location of max power window
- cross-correlation between the spectra

Similarly processed sub-signals from other original source signal(s)

(4 sub-signals)

113 — Calculate selective average

(4n sub-signals)

(1 sub-signal)

114 — Estimate respiratory rate

**FIG. 1A**

**200**

101 → Peripheral pulse wave

Peripheral pulse wave (other sensor)

102 → Low pass filter

104 → Detrend

104a → Smoothen

105 → Detect local maxima and minima

104b → Detect local maxima and minima

105a → For each maximum and minimum of the smoothened data, find the corresponding maxima and minima of the detrended data

105b → Find beat candidates

105c → Select best candidates

105d → Perform inter-beat-analysis

105e → Divide beat series into sub-signals

106a → Calculate puse wave widths (sub-signal 1)

106c → Calculate beat-to-beat intervals (sub-signal 3)

106b → Calculate pulse wave amplitudes (sub-signal 2)

106d → Calculate baseline variation (sub-signal 4)

107 → Resample at constant intervals (cubic interpolation)

106e → Calculate composite sub-signal (sub-signal 5)

106f → Calculate composite sub-signal (sub-signal 6)

**FIG. 1B**

Continues on next page

FIG. 1B

Raw data

**FIG. 2**

Signal 1  -pwv: pulse wave variability (resampled sub-signal 1)

**FIG. 3A**

18

Signal 2 -pav: pulse amplitude variability (resampled sub-signal 2)

**FIG. 3B**

Signal 3 -prv: pulse rate variability (resampled sub-signal 3)

**FIG. 3C**

Signal 4 -blv: baseline variability (resampled sub-signal 4)

**FIG. 3D**

**400**

**FIG. 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009016334 A